# EUROPEAN PATENT APPLICATION

(11) **EP 1 645 266 A1**
(43) Date of publication of application: **12.04.2006**
(21) Application number: 05021878.3
(22) Date of filing: 06.10.2005
(51) Int. Cl.: A61K 9/10, A61K 9/00, A61K 31/56, A61K 31/57, A61K 31/573, A61K 47/26, A61K 47/38, C07J 5/00, C07J 7/00, A61P 11/00, A61P 11/02

(54) **Aqueous suspension for nasal drops**

(30) Priority: 08.10.2004 JP 2004295537
(71) Applicant: Hisamitsu Medical Co., Ltd., Tokyo 100-6221 (JP)
(72) Inventor: Narui, Takashi, Sakura-shi Chiba 285-0817 (JP); Horie, Toshiaki, Katori-gun Chiba 287-0205 (JP)
(74) Representative: Hartz, Nikolai

(57) **Abstract**

It is intended to provide an aqueous suspension for nasal drops, which is easy to prepare, with maintaining superior long-term stability, good redispersibility and high retainability after the intranasal administration, as well as having good after feel.

The aqueous suspension for nasal drops comprising containing the following (a), (b), (c), (d), (e) and (f) :
(a) A compound represented by the following formula (1) 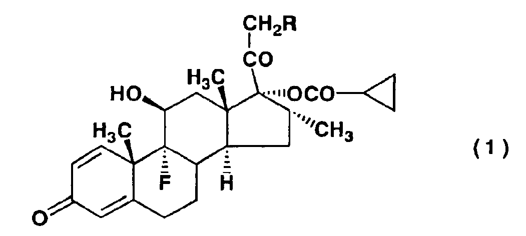 wherein R is hydroxyl or cyclohexylcarbonyloxy, or solvate thereof;
(b) crystalline cellulose-carmellose sodium 0.1 - 5% by weight;
(c) hydroxypropylcellulose 0.05 - 1% by weight;
(d) nonionic surfactant 0.001 - 0.2% by weight;
(e) moistening agent; and
(f) buffering agent.

## Description

### [Technical field]

The present invention relates to an aqueous suspension for nasal drops containing steroidal antiinflammatory agent as a main active ingredient with superior stability and redispersibility as well as retainability and good feeling after the intranasal administration.

### [Background art]

A steroid derivative represented by the following formula is a compound having strong local antiinflammatory action with reduced systemic adverse reaction through transdermal absorption (Patent document 1).

[0003] 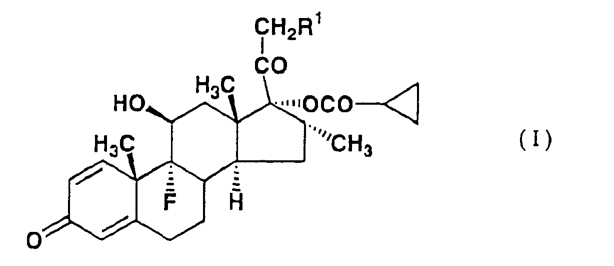 wherein R¹ is a group defined in the patent document 1.

The compound (1) has formulated as powders for inhalation useful for treatment of inflammatory respiratory tract disease such as bronchial asthma due to having highly selective local antiinflammatory action (Patent document 2). Recently, it is desired to develop aqueous liquid preparations for nasal drops aiming at prevention and treatment of nasal hypersensitivity such as allergic rhinitis and vasomotor rhinitis, and upper respiratory inflammatory disease such as sinusitis by utilizing the above compound.

A method using crystalline cellulose-carmellose sodium as a suspending agent is known for preparing aqueous liquid preparation of slightly soluble drugs (Patent documents 3 - 5). The liquid preparation, which is difficult to cause heterogeneity, can be obtained by addition of crystalline cellulose-carmellose sodium. However, since the heterogeneous state will occur in some drugs with time-dependent manner, a method adding further sodium alginate, carboxyvinyl polymer, sodium polyacrylate and sodium hyaluronate (Patent document 6), a method with preparing spherical form drug particles (Patent documents 7 and 8), and a method adding further nonionic cellulose ethers such as hydroxypropylcellulose, hydroxypropylmethylcellulose and hydroxyethylcellulose (Patent document 9) are proposed.

For preparing the compound of the formula (1) in the aqueous liquid suspension for nasal drops, the suspension dosage form has to be employed, since the compound (1) is extremely slightly soluble, and irritation of the preparation for applying to the nasal mucosa is preferably as low as possible, and solubilization of drug using large amount of surface active agent and organic solvent is quite difficult. Although crystalline cellulose-carmellose sodium is widely used as the suspending agent, the compound (1) is difficult to prepare in the form of suspension by using only crystalline cellulose-carmellose sodium. In addition, the aqueous suspension formula is often administered by using a spray-type constant volume spraying vessel for nasal drops, and in this case, aggregation and adhesion of fine particles will occur, and as a result, constant amount can not be sprayed.
[Patent document 1] JP,07-116215,B(1995)
[Patent document 2] JP,2004-18440,A
[Patent document 3] JP,10-259132,A(1998)
[Patent document 4] JP,2003-506396,A
[Patent document 5] JP,2004-503486,A
[Patent document 6] JP,11-130658,A(1999)
[Patent document 7] JP,11-130659,A(1999)
[Patent document 8] JP,2004-503489,A
[Patent document 9] JP,11-130660,A(1999)

### [Disclosure of the invention]

### [Problem to be solved by the invention]

It is an object of the present invention to provide an aqueous suspension for nasal drops, which is easy to prepare, possible to maintaining superior long-term stability, being sprayed in constant volume spray, having good redispersibility and high retainability after the intranasal administration, as well as having good after feel.

### [Means for solving the problem]

The inventors of the present invention have extensively studied a way to solve the problem and found that the aqueous liquid preparation, which is comprised of the compound represented by the following formula (1), salt thereof or solvate thereof suspended in a specific amount of crystalline cellulose-carmellose sodium and a specific amount of nonionic surfactant, moistening agent and buffering agent, can be easily prepared without exhibiting aggregation in preparation and decreased content of the compound, and with showing superior suspension stability, good redispersibility, less irritation to the nasal mucosa and long-term intramucosal retainability, and completed the present invention.

The present invention provides the aqueous suspension for nasal drops comprising containing the following (a), (b), (c), (d), (e) and (f):

(a) A compound represented by the following formula (1) 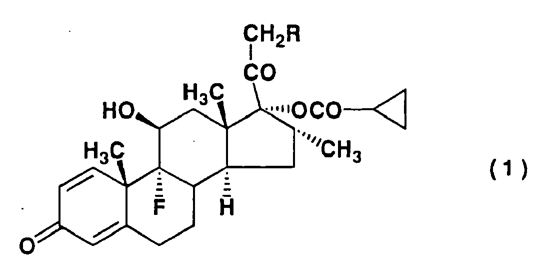 [0011] wherein R is hydroxyl or cyclohexylcarbonyloxy, or solvate thereof;
(b) crystalline cellulose-carmellose sodium 0.1 - 5% by weight;
(c) hydroxypropylcellulose 0.05 - 1% by weight;
(d) nonionic surfactant 0.001 - 0.2% by weight;
(e) moistening agent; and
(f) buffering agent.

### [Effect of the invention]

Since the aqueous suspension for nasal drops of the present invention can be prepared easily and sprayed with constant volume, in addition, exhibits no decrease in content of active ingredient, is superior in stability of suspension with good redispersibility, and exhibits less irritability to the nasal mucosa and long-term stability, it is superior in safety, stability, good after feel and productivity, and as a result, it is useful for preventive and therapeutic drug for nasal hypersensitivity such as allergic rhinitis and vasomotor rhinitis, and upper respiratory inflammatory disease such as sinusitis.

### [Best mode for carrying out the invention]

Examples of R in the compound represented by the formula (1) hereinbefore (hereinafter designates as the compound (1)) are hydroxyl or cyclohexylcarbonyloxy, and cyclohexylcarbonyloxy is preferable. The compound (1) may be a hydrate or a solvate attached with solvent used in the production and purification of the compound, for example water and alcohol.

The compound (1) is colorless crystal and can be produced by the method described in the specification of JP, 07-116215, B (1995). The compound (1) is preferably particles having mean diameter 20 µm, more preferably 7 µm or less, most preferably 5 µm or less.

Content of the compound (1) or solvate thereof in the aqueous suspension for nasal drops of the present invention is generally 0.01 - 1% by weight, preferably 0.05 - 0.5% by weight and most preferably 0.1 - 0.3% by weight to the total weight of the aqueous preparation.

Crystalline cellulose-carmellose sodium and hydroxypropylcellulose are added as the suspending agent to the aqueous suspension for nasal drops of the present invention. Crystalline cellulose-carmellose sodium is generally a mixture of crystalline cellulose content of 80% by weight or more and carmellose sodium content of 9 - 13% by weight, and, for example, can be available as Avicel RC-A591NF (Asahi Kasei Co.). Amount of crystalline cellulose-carmellose sodium to be added in the aqueous suspension for nasal drops of the present invention is 0.1 - 5% by weight, preferably 1 - 2% by weight to the total weight of the aqueous suspension preparation.

Hydroxypropylcellulose (HPC) contains generally hydroxypropoxyl group at 53 - 78%. Examples of HPC which can be available are products having various viscosities from extremely low viscosity to high viscosity measured by type B viscometer in 2% aqueous solution at 20°C, for example, viscosity 2.0 - 2.9 cps (e.g. Nisso HPC SSL Type, Nippon Soda Co.), viscosity 3.0 - 5.9 cps (e.g. Nisso HPC SL Type, Nippon Soda Co.), viscosity 6.0 - 10.0 cps (e.g. Nisso HPC L Type, Nippon Soda Co.), viscosity 150 - 400 cps (e.g. Nisso HPC M Type, Nippon Soda Co.) and viscosity 1000 - 4000 cps (e.g. Nisso HPC H Type, Nippon Soda Co.). One or two or more type selected therefrom can be used for the aqueous suspension for nasal drops of the present invention, and the HPC having viscosity 150 - 400 cps (e.g. Nisso HPC M Type, Nippon Soda Co.) is preferably used. Amount of HPC to be added to the aqueous suspension for nasal drops of the present invention depends on type of HPC, and is 0.05 - 1% by weight, preferably 0.1 - 0.5% by weight to the total weight of the aqueous suspension.

The compound (1) or solvate thereof exhibits pharmacological effect in extremely small amount, but it does not almost soluble in aqueous solvent and exhibits extremely worse wetting property. From the standpoint of improved dispersibility of the compound (1) at the preparation of the aqueous suspension for nasal drops, continuous stable dispersing condition, and improved redispersibility, nonionic surfactant is added to the aqueous suspension for nasal drops of the present invention. With regard to the nonionic surfactant, the surfactant having high suspending effect to the compound (1) is preferable. Examples of such the nonionic surfactant are polyoxyethylene (20) sorbitan monooleate and polyoxyethylene hydrogenated castor oil 60, and polyoxyethylene (20) sorbitan monooleate is especially preferable.

If amount of addition of the nonionic surfactant to the aqueous suspension for nasal drops of the present invention is small, aggregation of the compound (1) may occur at the preparation, resulting to decrease dispersibility, and as a result, homogeneous aqueous preparation can not be obtained. On the contrary, if amount of addition is too large, redispersibility may decrease and constant administration of the aqueous suspension may become difficult. Amount of addition of the nonionic surfactant depends on types of the nonionic surfactant. For example, amount of addition of polyoxyethylene (20) sorbitan monooleate is preferably 0.001 - 0.2% by weight, more preferably 0.005 - 0.1% by weight to the total weight of the aqueous suspension.

A moistening agent is generally added to the aqueous nasal drops for preventing dryness of the mucosa and irritation. The moistening agent having high suspending effect to the compound (1) is used in the aqueous suspension for nasal drops of the present invention. Examples of such the moistening agent are propylene glycol, glycerol, sorbitol, carboxyvinyl polymer, carmellose sodium, povidone, polyethylene glycol and agar or mixture thereof. Preferable example is propylene glycol or glycerol. Propylene glycol or glycerol can be used alone, and a combination thereof makes improved effect for suspending ability to the compound (1).

Amount of addition of the moistening agent is preferably 0.05 - 30% by weight, more preferably 0.1 - 5% by weight to total amount of the aqueous suspension for nasal drops of the present invention. In case of combination with propylene glycol and glycerol, amount of addition of propylene glycol to the aqueous suspension for nasal drops is preferably 0.05 - 20% by weight, more preferably 0.1 - 1% by weight, and amount of addition of glycerol is preferably 0.1 - 6% by weight, more preferably 1 - 4% by weight.

The buffering agent is further added to the aqueous suspension for nasal drops of the present invention. Preferable buffering agent is preferably not to inhibit suspension stability of the compound (1) and preferably to maintain the aqueous suspension at pH 5 - 7, which is less irritant to the nasal mucosa, more preferably to maintain at pH 6 - 7. Examples of such buffering agent are phosphate such as sodium hydrogenphosphate, potassium dihydrogenphosphate, dipotassium phosphate, anhydrous sodium dihydrogenphosphate, crystalline sodium dihydrogenphosphate; boric acid and borax; acetate such as sodium acetate; citric acid or citrate such as citric acid, citric anhydride, sodium citrate; amino acid salt such as sodium glutamate; and creatinine. Among them, phosphate is preferable, and sodium hydrogenphosphate and potassium dihydrogenphosphate are preferably used in combination. pH of the aqueous suspension for nasal drops of the present invention with using the buffering agent hereinabove is preferably adjusted at pH 5-7, more preferably pH 6 - 7.

Amount of addition of the buffering agent is preferably 0.005 - 2% by weight, more preferably 0.02 - 0.1% by weight to total amount of the aqueous suspension for nasal drops of the present invention. In case of combination with sodium hydrogenphosphate and potassium dihydrogenphosphate, amount of addition of sodium hydrogenphosphate is preferably 0.01 - 1% by weight, more preferably 0.03 - 0.04% by weight, and amount of addition of potassium dihydrogenphosphate is preferably 0.005 - 0.5% by weight, more preferably 0.02 - 0.03% by weight.

The preservative is preferably further added to the aqueous suspension for nasal drops of the present invention. Preferable preservative is preferably not to inhibit suspension stability of the compound (1) with fewer irritants to the nasal mucosa. Examples of such preservative are phenol such as phenol, benzyl alcohol, phenylethyl alcohol and chlorhexidine; quaternary ammonium compound such as benzalkonium chloride and benzethonium chloride; p-hydroxybenzoate such as methyl p-hydroxybenzoate, ethyl p-hydroxybenzoate, butyl p-hydroxybenzoate and propyl p-hydroxybenzoate; alcohol such as ethanol and chlorobutanol; mercury compound such as thimerosal; sodium dehydroacetate; and myristyl-gamma-picolinium chloride. One of such compound or two or more in combination thereof can be used. Among them, phenol (preferably phenylethyl alcohol) and/or quaternary ammonium compound (preferably benzalkonium chloride) are preferable, and a combination of phenylethyl alcohol and benzalkonium chloride is preferable.

Amount of addition of the preservative is preferably 0. 001 - 1% by weight, more preferably 0.04 - 0.4% by weight to total amount of the aqueous suspension for nasal drops of the present invention. In case of combination with phenylethyl alcohol and benzalkonium chloride, amount of addition of phenylethyl alcohol to the aqueous suspension for nasal drops is preferably 0.1 - 0.5% by weight, more preferably 0.2 - 0.3% by weight, and amount of addition of benzalkonium chloride is preferably 0.001 - 0.08% by weight, more preferably 0.004 - 0.006% by weight.

In the aqueous suspension for nasal drops of the present invention, amount of suspending agent, moistening agent, nonionic surfactant, buffering agent and preservative is adjusted to be within the range of osmotic pressure used for the aqueous suspension for nasal drops. Preferable osmotic pressure is equivalent to the osmotic pressure of aqueous sodium chloride solution 0.1 - 5% by weight, more preferably equivalent to the osmotic pressure of aqueous sodium chloride solution 0.5 - 1.5% by weight. If necessary, a tonicity adjusting agent such as sodium chloride, potassium chloride, mannitol, sorbitol, glucose and fructose may be added to the aqueous suspension for nasal drops of the present invention within a range not to inhibit stability of the compound (1).

Further, if necessary, stabilizing agent such as acetanilide, sodium hydrogensulfite, sodium pyrosulfite, dry sodium sulfite, sodium thiosulfate, disodium edetate, sodium citrate, ascorbic acid, nicotinic acid amide, hydrochloric acid, sodium hydroxide, potassium iodide, magnesium stearate, dibutylated hydroxytoluene, phenacetin, sodium propionate, butylated hydroxyanisole, propyl gallate and sodium formaldehydesulfoxylate, and refrigerant such as mentha water, mentha oil and 1-menthol may be added to the aqueous suspension for nasal drops of the present invention within a range not to inhibit stability of the compound (1).

The aqueous suspension for nasal drops of the present invention can be produced according to the known method. Namely, the compound (1), the suspending agent hereinabove and additives hereinabove are added to the pharmaceutically acceptable aqueous medium such as purified water and distilled water for injection to prepare homogeneous suspension by mixing propeller mixer, homomixer, homogenizer, etc.

The aqueous suspension for nasal drops of the present invention is administered to the intranasal cavity by means of a method such as spraying and dropping used in the general nasal drops. For example, in the spraying method, the aqueous suspension for nasal drops of the present invention is sprayed once or twice a day, in a dose of about 25 - 200 µl, to the nasal cavities by using sprayer. In the dropping method, the aqueous suspension for nasal drops of the present invention may be dropped from the nostril. Dosage may be changed ad libitum depending on age, body weight and symptoms.

### Examples

The present invention is further explained in detail by illustrating examples hereinbelow, but the present invention is not limited by such illustrating examples.

### Example 1

A compound (1a) (in the formula (1), R = cyclohexylcarbonyloxy group) (average particle diameter 2.35 µm), crystalline cellulose-carmellose sodium (Avicel RC-A591NF, Asahi Kasei Co.), hydroxypropylcellulose (Nisso HPC M Type, Nippon Soda Co.), polyoxyethylene (20) sorbitan monooleate (TO-10MV, Nikko Chemicals Co.), propylene glycol, glycerol, sodium hydrogenphosphate, potassium dihydrogenphosphate, phenylethyl alcohol and 10% benzalkonium chloride solution were weighted according to amount of the prescription in Table 1, and added to the purified water 90 g and stirred for 30 minutes using homomixer (6000 rpm). To the obtained suspension was added the purified water to be total amount 100 g, and further stirred for 10 minutes to prepare the aqueous suspension for nasal drops as shown in examples 1-1 to 1-5, and comparative examples 1-1 to 1-2.

**Table 1**

| Amount of Prescription (g). | Example | | | | | Comparative example | |
|---|---|---|---|---|---|---|---|
| | 1-1 | 1-2 | 1-3 | 1-4 | 1-5 | 1-1 | 1-2 |
| Compound (1a) | 0.28 | 0.28 | 0.28 | 0.28 | 0.28 | 0.28 | 0.28 |
| Crystalline cellulose-carmellose sodium | 5.0 | 2.0 | 1.5 | 1.0 | 0.1 | 0 | 6.0 |
| Hydroxypropylcellulose (HPC-M) | 0.25 | 0.25 | 0.25 | 0.25 | 0.25 | 0.25 | 0.25 |
| Polyxoyethylene (20) sorbitan monooleate | 0.01 | 0.01 | 0.01 | 0.01 | 0.01 | 0.01 | 0.01 |
| Glycerol | 2.5 | 2.5 | 2.5 | 2.5 | 2.5 | 2.5 | 2.5 |
| Propylene glycol | 0.5 | 0.5 | 0.5 | 0.5 | 0.5 | 0.5 | 0.5 |
| Sodium hydrogenphosphate | 0.035 | 0.035 | 0.035 | 0.035 | 0.035 | 0.035 | 0.035 |
| Potassium dihydrogenphosphate | 0.022 | 0.022 | 0.022 | 0.022 | 0.022 | 0.022 | 0.022 |
| Phenylethyl alcohol | 0.25 | 0.25 | 0.25 | 0.25 | 0.25 | 0.25 | 0.25 |
| 10% benzalkonium chloride solution | 0.05 | 0.05 | 0.05 | 0.05 | 0.05 | 0.05 | 0.05 |
| Sterile purified water | q.s. | q.s. | q.s. | q.s. | q.s. | q.s. | q.s. |
| Total | 100 | 100 | 100 | 100 | 100 | 100 | 100 |

### Test example 1

The aqueous suspension for nasal drops of examples 1-1 to 1-5 and comparative examples 1-1 to 1-2 was packed into the spray type polyethylene vessel for nasal drop (10 ml) . The suspension stability test, the redispersibility test and the spray test were performed according to the method hereinbelow. Results are shown in Table 2.

### Dispersibility at preparation:

Dispersion of the aqueous suspension for nasal drops at the time of preparation was indicated by a mark: **"●:** can be easily dispersed", "o: can be dispersed" and "×: aggregation was observed".

### Suspension stability test:

A volume of 0.5 ml of the aqueous suspension for nasal drops packed in the vessel for nasal drop was collected immediately after packing and after storage at 20°C for 24 hours from the top surface of the liquid and the basal plane of the liquid. Content of the compound (1a) was measured (n=3) by using HPLC. A ratio to the prescribed amount is shown by %.

### Redispersibility test:

Redispersibility of the aqueous suspension for nasal drops packed in the vessel for nasal drop after storage at 40°C for 3 months was indicated by a mark: "o: redispersion was observed by numbers of reverse rotation as shown in the parentheses" and "×: no redispersion was observed by 20 reverse rotations".

### Spraying test:

The aqueous suspension for nasal drops packed in the vessel for nasal drop was sprayed for 15 consecutive days, once a day, twice a spray time. Appearance of the nozzle after the spray was indicated by: "o: no adhesive was observed"; "▲: adhesive was slightly observed"; and "×: adhesive was observed".

**Table 2**

| | | | Example | | | | | Comparative example | |
|---|---|---|---|---|---|---|---|---|---|
| | | | 1-1 | 1-2 | 1-3 | 1-4 | 1-5 | 1-1 | 1-2 |
| Dispersibility at preparation | | | ● | ● | ● | ● | ● | o | ● |
| Suspension stability test | At packing | Top surface | 99.2 | 99.2 | 99.2 | 100.3 | 99.2 | 100.1 | 100.0 |
| | | Basal plane | 99.3 | 99.3 | 99.3 | 100.2 | 99.6 | 100.3 | 100.1 |
| | After 24 hrs. | Top surface | 99.2 | 99.2 | 99.2 | 99.5 | 99.4 | 95.9 | 99.4 |
| | | Basal plane | 99.2 | 99.2 | 99.2 | 100.1 | 99.6 | 103.6 | 99.3 |
| Redispersibility test | | | o(1) | o(1) | o(1) | o(3) | o(8) | × | o(1) |
| Spraying test | | | ▲ | o | o | o | o | o | × |

### Example 2

The aqueous suspension for nasal drops of examples 2-1 to 2-4 and comparative examples 2-1 to 2-2 were prepared using the prescribed amount as shown in Table 3 by the same way as in example 1.

**Table 3**

| Amount of Prescription (g) | Example | | | | Comparative example | |
|---|---|---|---|---|---|---|
| | 2-1 | 2-2 | 2-3 | 2-4 | 2-1 | 2-2 |
| Compound (1a) | 0.28 | 0.28 | 0.28 | 0.28 | 0.28 | 0.28 |
| Crystalline cellulose-carmellose sodium | 1.5 | 1.5 | 1.5 | 1.5 | 1.5 | 1.5 |
| Hydroxypropyl cellulose (HPC-M) | 1 | 0.5 | 0.1 | 0.05 | 0 | 2 |
| Polyxoyethylene (20) sorbitan monooleate | 0.01 | 0.01 | 0.01 | 0.01 | 0.01 | 0.01 |
| Glycerol | 2.5 | 2.5 | 2.5 | 2.5 | 2.5 | 2.5 |
| Propylene glycol | 0.5 | 0.5 | 0.5 | 0.5 | 0.5 | 0.5 |
| Sodium hydrogenphosphate | 0.035 | 0.035 | 0.035 | 0.035 | 0.035 | 0.035 |
| Potassium dihydrogenphosphate | 0.022 | 0.022 | 0.022 | 0.022 | 0.022 | 0.022 |
| Phenylethyl alcohol | 0.25 | 0.25 | 0.25 | 0.25 | 0.25 | 0.25 |
| 10% benzalkonium chloride solution | 0.05 | 0.05 | 0.05 | 0.05 | 0.05 | 0.05 |
| Sterile purified water | q.s. | q.s. | q.s. | q.s | q.s, | q.s. |
| Total | 100 | 100 | 100 | 100 | 100 | 100 |

### Test example 2

The aqueous suspension for nasal drops of examples 2-1 to 2-4 and comparative examples 2-1 to 2-2 was packed into the spray type polyethylene vessel for nasal drop (10 ml). The suspension stability test, the redispersibility test and the spray test were performed by the same way as in test example 1. Results are shown in Table 4.

**Table4**

| | | | Example | | | | Comparative example | |
|---|---|---|---|---|---|---|---|---|
| | | | 2-1 | 2-2 | 2-3 | 2-4 | 2-1 | 2-2 |
| Dispersibility at preparation | | | ● | ● | ● | ● | o | ● |
| Suspension stability test | At packing | Top surface | 99.2 | 99.4 | 99.6 | 100.1 | 100.6 | 100.5 |
| | | Basal plane | 99.3 | 99.6 | 99.6 | 100.2 | 100.6 | 100.6 |
| | After 24 hrs | Top surface | 99.2 | 99.3 | 99.4 | 99.9 | 96.5 | 100.4 |
| | | Basal plane | 99.2 | 99.4 | 99.2 | 100.5 | 102.0 | 100.7 |
| Redispersibility test | | | o(1) | o(1l) | o(1) | o(3) | o(18) | o(1) |
| Spraying test | | | ▲ | o | o | o | o | × |

### Example 3

The aqueous suspension for nasal drops of examples 3-1 to 3-4 and comparative examples 3-1 to 3-2 were prepared using the prescribed amount as shown in Table 5 by the same way as in example 1.

**Table 5**

| Amount of Prescription (g) | Example | | | | Comparative example | |
|---|---|---|---|---|---|---|
| | 3-1 | 3-2 | 3-3 | 3-4 | 3-1 | 3-2 |
| Compound (1a) | 0.28 | 0.28 | 0.28 | 0.28 | 0.28 | 0.28 |
| Crystalline cellulose-carmellose sodium | 1.5 | 1.5 | 1.5 | 1.5 | 1.5 | 1.5 |
| Hydroxypropylcellulose (HPC-M) | 0.25 | 0.25 | 0.25 | 0.25 | 0.25 | 0.25 |
| Polyxoyethylene (20) sorbitan monooleate | 0.2 | 0.1 | 0.005 | 0.001 | 0 | 0.4 |
| Glycerol | 2.5 | 2.5 | 2.5 | 2.5 | 2.5 | 2.5 |
| Propylene glycol | 0.5 | 0.5 | 0.5 | 0.5 | 0.5 | 0.5 |
| Sodium hydrogenphosphate | 0.035 | 0.035 | 0.035 | 0.035 | 0.035 | 0.035 |
| Potassium dihydrogenphosphate | 0.022 | 0.022 | 0.022 | 0.022 | 0.022 | 0.022 |
| Phenylethyl alcohol | 0.25 | 0.25 | 0.25 | 0.25 | 0.25 | 0.25 |
| 10% benzalkonium chloride solution | 0.05 | 0.05 | 0.05 | 0.05 | 0.05 | 0.05 |
| Sterile purified water | q.s. | q.s. | q.s. | q.s. | q.s. | q.s. |
| Total | 100 | 100 | 100 | 100 | 100 | 100 |

### Test example 3

The aqueous suspension for nasal drops of examples 3-1 to 3-4 and comparative examples 3-1 to 3-2 was packed into the spray type polyethylene vessel for nasal drop (10 ml). The suspension stability test, the redispersibility test and the spray test were performed by the same way as in test example 1. Results are shown in Table 6.

**Table6**

| | | | Example | | | | Comparative example | |
|---|---|---|---|---|---|---|---|---|
| | | | 3-1 | 3-2 | 3-3 | 3-4 | 3-1 | 3-2 |
| Dispersibility at preparation | | | ● | ● | ● | o | × | ● |
| Suspension stability test | At packing | Top surface | 100.8 | 99.5 | 100.0 | 100.4 | - | 99.1 |
| | | Basal plane | 100.6 | 99.6 | 100.2 | 100.5 | - | 99.3 |
| | After 24 hrs | Top surface | 100.5 | 99.8 | 99.9 | 100.2 | - | 99.3 |
| | | Basal plane | 100.5 | 99.6 | 100.0 | 100.3 | - | 99.3 |
| Redispersibility test | | | o(3) | o(1) | o(1) | o(1) | - | o(7) |
| Spraying test | | | o | o | o | o | - | o |

### Example 4

The aqueous suspension for nasal drops of examples 4-1 to 4-3 and comparative examples 4-1 to 4-2 were prepared by the same way as in example 1 using the prescribed amount as shown in Table 7.

**Table 7**

| Amount of Prescription (g) | Example | | | Comparative example | |
|---|---|---|---|---|---|
| | 4-1 | 4-2 | 4-3 | 4-1 | 4-2 |
| Compound (1a) | 0.14 | 0.01 | 1.0 | 0.14 | 0.14 |
| Crystalline cellulose-carmellose sodium | 1.5 | 1.5 | 1.5 | 1.5 | 1.5 |
| Hydroxypropylcellulose (HPC-M) | 0.25 | 0.25 | 0.25 | 0.25 | 0.25 |
| Polyxoyethylene (20) sorbitan monooleate | 0.01 | 0.01 | 0.01 | 0.01 | 0.01 |
| Glycerol | 0.5 | 0.5 | 0.5 | 0 | 0.5 |
| Propylene glycol | 2.5 | 2.5 | 2.5 | 0 | 2.5 |
| Sodium hydrogenphosphate | 0.035 | 0.035 | 0.035 | 0.035 | 0 |
| Potassium dihydrogenphosphate | 0.022 | 0.022 | 0.022 | 0.022 | 0 |
| Phenylethyl alcohol | 0.25 | 0.25 | 0.25 | 0.25 | 0.25 |
| 10% benzalkonium chloride solution | 0.05 | 0.05 | 0.05 | 0.05 | 0.05 |
| Sterile purified water | q.s. | q.s. | q.s. | q.s. | q.s. |
| Total | 100 | 100 | 100 | 100 | 100 |

### Test example 4

The aqueous suspension for nasal drops of examples 4-1 to 4-3 and comparative examples 4-1 to 4-2 was packed into the spray type polyethylene vessel for nasal drop (10 ml). The suspension stability test, the redispersibility test and the spray test were performed by the same way as in test example 1. Results are shown in Table 8.

**Table8**

| | | | Example | | | Comparative example | |
|---|---|---|---|---|---|---|---|
| | | | 4-1 | 4-2 | 4-3 | 4-1 | 4-2 |
| Dispersibility at preparation | | | ● | ● | ● | ● | ● |
| Suspension stability test | At packing | Top surface | 99.6 | 100.7 | 99.9 | 99.8 | 100.2 |
| | | Basal plane | 99.5 | 100.6 | 99.9 | 99.5 | 99.5 |
| | After 24 hrs | Top surface | 99.4 | 100.7 | 100.1 | 99.2 | 99.4 |
| | | Basal plane | 99.2 | 101.0 | 100.1 | 99.4 | 99.7 |
| Redispersibility test | | | o(1) | o(1) | o(2) | o(1) | o(1) |
| Spraying test | | | o | o | o | o | o |

### Test example 5

### Test for feeling after use

The aqueous suspensions for nasal drops of examples 1-1, 4-1 and comparative examples 4-1 to 4-2 packed in the spray type polyethylene vessel for nasal drop (10 ml) were used. Each aqueous suspension for nasal drops 100 µl was sprayed once into the nasal cavity of 5 volunteers. Test for feeling after use in the volunteers was performed by observing the numbers of persons who exhibited "liquid dripping" and "irritation". Results indicating the numbers of persons are shown in Table 9.

**Table 9**

| | | Example | | Comparative example | |
|---|---|---|---|---|---|
| | | 1-1 | 4-1 | 4-1 | 4-2 |
| Feeling after use | Liquid dripping | 0 | 0 | 2 | 0 |
| | Irritation | 0 | 0 | 0 | 3 |

## Claims

1. An aqueous suspension for nasal drops comprising containing the following (a), (b), (c), (d), (e) and (f) :
(a) A compound represented by the following formula (1) 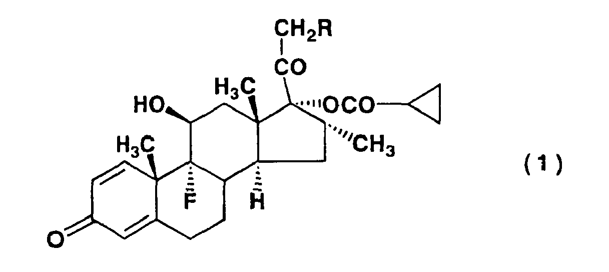 wherein R is hydroxyl or cyclohexylcarbonyloxy, or solvate thereof;
(b) crystalline cellulose-carmellose sodium 0.1 - 5% by weight;
(c) hydroxypropylcellulose 0.05 - 1% by weight;
(d) nonionic surfactant 0.001 - 0.2% by weight;
(e) moistening agent; and
(f) buffering agent.

2. The aqueous suspension for nasal drops according to claim 1 comprising the compound represented by the formula (1) or solvate thereof 0.01 - 1% by weight, the moistening agent 0.05 - 30% by weight, the buffering agent 0.005 - 2% by weight and a preservative 0.001 - 1% by weight.

3. The aqueous suspension for nasal drops according to claim 1 comprising the compound represented by the formula (1) or solvate thereof 0.1 - 0.3% by weight, crystalline cellulose-carmellose sodium 1 - 2% by weight, hydroxypropylcellulose 0.1 - 0.5% by weight, nonionic surfactant 0.005 - 0.1% by weight, the moistening agent 0.1 - 5% by weight, the buffering agent 0 . 02 - 0.1% by weight and the preservative 0.04 - 0.4% by weight.

4. The aqueous suspension for nasal drops according to any of claims 1 to 3 wherein the average particle size of the compound represented by the formula (1) or solvate thereof is 20 µm or less.

5. The aqueous suspension for nasal drops according to any of claims 1 to 4 wherein the average particle size of the compound represented by the formula (1) or solvate thereof is 5 µm or less.

6. The aqueous suspension for nasal drops according to any one of claims 1 to 5 wherein the viscosity of 2% aqueous solution of hydroxypropylcellulose is 150 - 400 cps when using the type B viscometer at 20°C.

7. The aqueous suspension for nasal drops according to any one of claims 1 to 6 wherein the nonionic surfactant is selected from the group consisting of polyoxyethylene (20) sorbitan monooleate and polyoxyethylene hydrogenated castor oil 60.

8. The aqueous suspension for nasal drops according to any one of claims 1 to 7 wherein the nonionic surfactant is polyoxyethylene (20) sorbitan monooleate.

9. The aqueous suspension for nasal drops according to any one of claims 1 to 8 wherein the moistening agent is one or more substance selected from the group consisting of glycerol, propylene glycol, sorbitol, carboxyvinyl polymer, carmellose sodium, povidone, polyethylene glycol and agar.

10. The aqueous suspension for nasal drops according to any one of claims 1 to 9 wherein the moistening agent is glycerol 0.1 - 6% by weight and propylene glycol 0.05 - 20% by weight.

11. The aqueous suspension for nasal drops according to any one of claims 1 to 10 wherein the moistening agent is glycerol 1 - 4% by weight and propylene glycol 0.1 - 1% by weight.

12. The aqueous suspension for nasal drops according to any one of claims 1 to 11 wherein the buffering agent is one or more substance selected from the group consisting of sodium hydrogenphosphate, potassium dihydrogenphosphate, dipotassium phosphate, anhydrous sodium dihydrogenphosphate, crystalline sodium dihydrogenphosphate, boric acid, borax, sodium acetate, citric acid, citric anhydride, sodium citrate, sodium glutamate and creatinine.

13. The aqueous suspension for nasal drops according to any one of claims 1 to 12 wherein the buffering agent is sodium hydrogenphosphate 0.01 - 1% by weight and potassium dihydrogenphosphate 0.005 - 0.5% by weight.

14. The aqueous suspension for nasal drops according to any one of claims 1 to 13 wherein the buffering agent is sodium hydrogenphosphate 0.03 - 0.04% by weight and potassium dihydrogenphosphate 0.02 - 0.03% by weight.

15. The aqueous suspension for nasal drops according to any one of claims 2 to 14 wherein the preservative is one or more substance selected from the group consisting of phenol, benzyl alcohol, phenylethyl alcohol, chlorhexidine, benzalkonium chloride, benzethonium chloride, methyl p-hydroxybenzoate, ethyl p-hydroxybenzoate, butyl p-hydroxybenzoate, propyl p-hydroxybenzoate, ethanol, chlorobutanol, thimerosal, sodium dehydroacetate and myristyl-gamma-picolinium chloride.

16. The aqueous suspension for nasal drops according to any one of claims 2 to 15 wherein the preservative is phenylethyl alcohol 0.1 - 0.5% by weight and benzalkonium chloride 0.001 - 0.08% by weight.

17. The aqueous suspension for nasal drops according to any one of claims 2 to 16 wherein the preservative is phenylethyl alcohol 0.2 - 0.3% by weight and benzalkonium chloride 0.004 - 0.006% by weight as benzalkonium chloride solution.

18. The aqueous suspension for nasal drops according to any one of claims 1 to 17 wherein pH of the aqueous suspension is pH 5 - 7.

19. The aqueous suspension for nasal drops according to any one of claims 1 to 18 wherein pH of the aqueous suspension is pH 6 - 7.
